# EUROPEAN PATENT SPECIFICATION

(11) **EP 4 326 155 B1**
(45) Date of publication and mention of the grant of the patent: **17.09.2025**
(21) Application number: 22722347.6
(22) Date of filing: 13.04.2022
(51) Int. Cl.: A61B 6/00, A61B 6/10, A61B 6/42

(54) **DEVICE AND METHOD FOR DETECTING AND MONITORING THE POTASSIUM CONCENTRATION**
VORRICHTUNG UND VERFAHREN ZUR DETEKTION UND ÜBERWACHUNG DER KALIUMKONZENTRATION
DISPOSITIF ET PROCÉDÉ DE DÉTECTION ET DE SURVEILLANCE DE LA CONCENTRATION EN POTASSIUM

(30) Priority: 19.04.2021 IT 202100009809
(43) Date of publication of application: 28.02.2024
(73) Proprietor: NG Detectors S.r.l., 00198 Roma (IT)
(72) Inventor: PERGOLA, Andrea, 00198 Roma (IT); PANI, Arianna, 00198 Roma (IT)
(74) Representative: Leone, Mario
(86) International application number: PCT/IB2022/053474
(87) International publication number: WO 2022/224088

(56) References cited:
- EP-A1- 2 909 654
- EP-B1- 2 909 654
- US-A1- 2012 273 687
- SASCHA MOEHRS ET AL: "A detector head design for small-animal PET with silicon photomultipliers (SiPM)", PHYSICS IN MEDICINE AND BIOLOGY, INSTITUTE OF PHYSICS PUBLISHING, BRISTOL GB, vol. 51, no. 5, 7 March 2006 (2006-03-07), pages 1113 - 1127, XP020096158, ISSN: 0031-9155, DOI: 10.1088/0031-9155/51/5/004

## Description

The present invention generally relates to a device for detecting radionuclides and a method for detecting and monitoring the Potassium concentration in a source using said detecting device.

As it is known, Potassium in nature consists of several isotopes: ³⁹K, ⁴⁰K e ⁴¹K. This composition is well defined and stable all over the planet Earth. ⁴⁰K, although existing in a very low percentage (about 0.012%) is a radionuclide and it is the only radioactive isotope of Potassium; it has an overall half-life in the order of one billion years which decays in a daughter nucleus through two possible decay processes which determine an emission of radiations.

In particular, ⁴⁰K is not produced by natural processes or other decay phenomena, and then it is called primordial, since it formed together with the solar system. Notoriously, it has two radioactive decay types: one through β-decay and the other one through electronic capture, causing a radiation emission y of 1460 keV.

As a consequence of these premises, an amount of 1 g of Potassium produces on the average 30 decays per second, and the decays which can be detected in a source represent the Potassium amount and then the concentration in that source.

Generally, instruments and methods for detecting and monitoring radionuclides are known and, in particular, spectrometers of gamma rays are used in a wide variety of applications, for example to identify and monitor gamma rays in applications of scientific, industrial and environmental monitoring, for example for medical purposes, for safety screening of personnel and goods, for example in border crossings, or for searching and evaluating orphan radioactive sources.

In particular, the emission of radionuclides can identify electrolyte imbalances being indicia of several pathologies. In fact, as it is known, the electrolyte disorders, such as the Potassium imbalance caused by different conditions, can be frequent, in particular in older population.

Hyperkalaemia and hypokalaemia are considered life-threatening electrolyte disorders: the excretion of Potassium can be reduced in case of diseases such as heart failure, hypertension, chronic and acute kidney damage which can appear more often with age.

Moreover, polypharmacy, that is the concomitant intake of five or more drugs, is a frequent condition in the sick person affected by several comorbidities and often it is associated to a hyperkalaemia condition. The use of drugs which can cause hyperkalaemia can be another risk factor in presence of kidney failure. A relation between Potassium imbalances, morbidity and mortality in older people was observed. For example, hyperkalaemia in this population can increase the need for haemodialysis, mechanical ventilation, hospitalization and follow-up in intensive care. All this can increase health costs and can have serious economic repercussions especially in the developing countries.

Hyperkalaemia, or hypopotassaemia, is a common clinical problem. Potassium enters the body through oral assumption or intravenous infusion, it is mostly stored in the cells and then it is excreted in the urine. The main causes of hyperkalaemia are the increase in the Potassium release from cells and, very often, the reduced urinary excretion of Potassium. Besides, a decrease in the assumption, an increase in the translocation into cells or, very often, an increase in the losses in urine, in the gastrointestinal tract or in the sweat can lead to a decrease in the Potassium serum concentration.

The most serious manifestations of hyperkalaemia and hypokalaemia are weakness or muscle paralysis, anomalies of cardiac conduction and cardiac arrhythmias, including sinus bradycardia, sinus arrest, idioventricular rhythms, ventricular tachycardia, ventricular fibrillation and asystole. These manifestations usually appear when the Potassium serum concentration is higher or equal to 7.0 mEq/L with chronic hyperkalaemia, or possibly at lower levels in patients with an acute increase in serum Potassium and/or the underlying cardiac conduction disease.

Potassium is classified in human being as intracellular and extracellular Potassium. The well-defined ratio is by a factor 40 in favour of the intracellular Potassium, which is responsible for the metabolic activities.

Currently, the evaluation of Potassium is performed by blood test (then test of the extracellular Potassium) by using an ion-selective electrode (ISE) converting the activity (or real concentration) of ions dissolved in solution into an electrical potential measured by a voltmeter.

Therefore, when one analyses the extracellular Potassium, one performs an indirect measurement of the Potassium amount first of all, and the measured amounts are very small and often the measurement precision fails by causing the high incidence of pseudo-hyperkalaemia and pseudo-normokalaemia phenomena. The reliability and precision in monitoring the Potassium serum level are an essential issue.

Clinical studies have demonstrated that there is a significant variability and inaccuracy in the Potassium plasmatic levels measured in whole blood samples.

Then there are two main problems in the real evaluation of the Potassium levels, first one among all the measurement inaccuracy, which can lead even to an underestimation of the phenomenon or to an inappropriate therapeutic intervention and, second but not least, the obligation on patient to reach physically an analysis laboratory with very discomfort and long waits or however, even in the luckiest cases, where the sample collection is performed at one's home, the limitation in performing the Potassium evaluation to a medical prescription which takes place on a one-off basis.

Another application for instruments detecting radionuclides relates to radioprotection in laboratories which use radioactive substances or in the nuclear plants in which measurements are regularly performed, for example to establish the radioactive contamination and to measure the radioactive activity.

US patent application No. 2016/0,299,251 A1, for example, relates to a system which detects the radiations of objects, materials, etc. existing in the environment, in particular in the ground, with the purpose of detecting the optimum points in order to proceed with drilling the ground to reach pockets of oil, gas or other fossil fuels.

US patents No. 4,590,377 A and No. 5,939,723 A relate to devices used in the field of slaughtering of animal meat, to monitor Potassium with the purpose of evaluating the leanness of the meat, since the fat content and the Potassium amount are correlated amounts in living organisms. The first one uses a device with a probe which has to be inserted in the meat after slaughtering, and the second one, instead, is used to monitor the amount of muscle fat in the living animal.

European patent No. EP 2,909,654 A1 relates to a portable gamma-chamber which integrates a display for displaying instantaneously the distribution of the radionuclides in the organism, the presence thereof is induced by radio drugs or radiotherapy, or within scintigraphies or similar *imaging* techniques.

Even US patent application No. 2012/0,273,687 A1 relates to a system of medical imaging equipped with a panel for dissipating heat and improving to display the results.

### SUMMARY OF THE INVENTION

The technical problem underlying the present invention is to provide a device for monitoring radionuclides and a relative method for applying said device, allowing to obviate the drawback mentioned with reference to the known art.

Such problem is solved by a detecting device for monitoring electrolytes in patients and a method as above specified and as defined in the respective enclosed independent claims.

### SUMMARY OF THE DISCLOSURE

The detecting device for monitoring the Potassium concentration according to the disclosure comprises a plurality of scintillation sensors, arranged to detect electromagnetic radiations between 20 keV and 2000 keV emitted by the decay of isotope ⁴⁰K, consisting of a first reference scintillation sensor, which is fully shielded, since it is enclosed in a shielding layer, and of second scintillation sensors, which are only partially shielded so as to achieve in each one a not receiving portion and a receiving portion.

The device further comprises a casing wherein a plurality of seats for scintillation sensors is formed, which are separate and distinct to each other, but connected. Each seat receives a respective sensor and the casing, as a whole, is deformable so that the position of each sensor could vary with respect to the position of other sensors, for example by bending or curving the casing as a whole.

In the casing, the position of the second sensors in the respective seats is so that the receiving portions thereof are oriented so as to determine together a common receiving surface in the detecting device, and wherein the casing can be adapted to fit the shape of said receiving surface to an emitting surface with irregular shape.

The main advantage of the device according to the present disclosure lies in the fact that it can be adapted to emitting surfaces with even irregular and changing shapes, such as for example a human body portion corresponding to an organ whose Potassium level has to be monitored.

The organs which contain more Potassium are liver, heart and brain: advantageously, said device could have sizes comparable with those of the right lobe of an adult liver or of an adult heart, and the receiving surface could adhere to the body at these organs to optimize the detection of the radiations emitted by the radionuclide of Potassium.

The same type of analysis could be performed even on other types of organic or inorganic, animal or vegetable, living or not living sources, the content of Potassium and/or its possible evolution over time thereof one wishes to estimate, by adapting the device to the shape of the source for a better evaluation precision, such as for example in one of the previously described examples, such as the one related to the determination of the amount of fat in the slaughtered meat.

Therefore, according to the present concept, a method for detecting and monitoring the Potassium concentration in a source with possibly irregular and/or variable shape, comprises a preparation step, wherein at least a device detecting electromagnetic radiations emitted by the decay of Potassium isotope ⁴⁰K is provided, comprising a plurality of scintillation sensors, wherein the detecting device is deformable to adapt it to the source surface and to optimize the evaluation of the Potassium content.

The method then will include an application step, wherein the shape of the detecting device is adapted to an emitting surface with a possibly irregular shape of the source, so as to establish a correlation between the emitting surface and a capture area of the detecting device; and a subsequent measurement step wherein a measurement of the Potassium concentration is performed, by obtaining it from the amount of electromagnetic radiations emitted by the decay of Potassium isotope ⁴⁰K. It will further be noted that the use of this detecting and monitoring method on a living animal organism, and in particular on a human being, requires that a correlation between the emitting surface and the receiving surface of the device is established, this correlation without necessarily involving a contact. The device, in fact, could be worn and it will be adhered to a layer of tissue worn by a patient, or it could be arranged in a suitable pocket, then adhered to the patient's body, or still placed in correlation thereto by means of a simple epidermal contact which does not alter the organism.

By applying the above-defined monitoring method, then it will be possible to establish an alarm threshold based upon the detected measurement of the Potassium concentration existing in the environment or in any emitting, in case animal or living, organism.

In particular, it will be possible to establish an alarm threshold based upon the exact measurement of the concentration of electrolytes in patients, by measuring exactly the intracellular Potassium concentration in the human organism and its variation over time, by performing periodical detections according to a predetermined protocol.

The present disclosure will be described hereinafter according to a preferred embodiment thereof, provided by way of example and not for limitative purposes with reference to the enclosed drawings wherein:
* figure 1 shows a flow diagram illustrating an embodiment example of the detecting and monitoring method according to the invention;
* figure 2A shows a perspective view of a first embodiment example of detecting device according to the invention;
* figure 2B shows a perspective view of a second embodiment example of detecting device according to the invention;
* figure 3 shows a partial view, in longitudinal section, of the device of figure 2A.

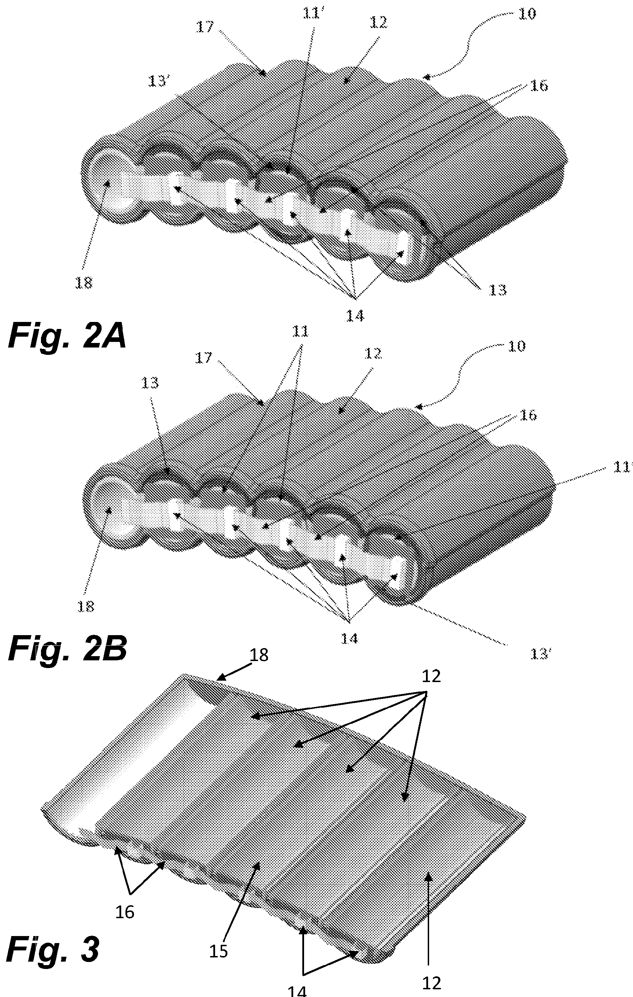

With reference to figures 2A and 2B, two examples of a device 10 detecting electromagnetic radiations emitted by the Potassium decay are described.

They include a plurality of sensors, consisting of a first scintillation sensor 11', which is shielded, that is it is wholly enclosed in a first shielding layer 13 which covers it completely.

Moreover, in said plurality of sensors, second scintillation sensors 11 are provided, which are in case equal to the first sensor 11', and which instead are partially shielded by a second shielding layer 13', so as to shield only a not receiving portion thereof and to leave uncovered a receiving portion thereof.

The shielding layer, both for the first and for the second sensor 11, 11' can consist in a layer of lead having a suitable thickness.

The scintillation sensors 11, 11' have a substantially cylindrical and longitudinally elongated shape, as far as an exemplifying length of about 10 cm.

The detecting device 10 even comprises a casing 12 (figure 3), substantially equal for both embodiments of figures 2A and 2B, wherein a plurality of seats for scintillation sensors which are separate and distinct, but connected to each other, is formed. Each seat receives a respective scintillation sensor 11', 11 and locks it in a predetermined position.

Moreover, the casing 12 is deformable, that is for example foldable, so that the position of each scintillation sensor 11, 11' could vary with respect to the position of others.

The position of the second sensors, or of their receiving portions is so that all together they are oriented so as to determine a receiving surface or face of the detecting device 10, and the casing 12 can be adapted to fit the shape of said receiving surface to a contact surface with irregular shape, in case variable over time.

The sensors 11, 11' are conveniently arranged placed side by side by minimizing their distance and, in this way, sensors 11, 11' form an area for capturing the radioactive emissions having predetermined width, and compatible with the intended purposes.

The seats then have a tubular shape, which conjugates with the shape of the sensors 11, 11' and locks them in a prefixed position.

Such seats include a plurality of external walls 17 which conjugate to each other to form the flexible casing 12 for all sensors 11, 11'.

In these examples, the casing 12 has a substantially cartridge case-like shape, and generally the above-mentioned seats are defined by continuous walls 17, which adhere to the shape of the external surface of the sensors 11, 11' and which are conjugated to each other through flexible diaphragms, to make the casing 12 deformable and foldable, in case even in flexible way.

Preferably, the casing 12 is made of a flexible, deformable and hypoallergenic material, such as silicon.

Alternatively, it could be made of a material comprised in the group consisting of: tissue, leather or any flexible and deformable elastomeric material.

The casing 12 is closed at the ends of the seats, in case with a re-closable opening.

In these examples, when the casing 12 is stretched out in a flat configuration, the longitudinal axes of the seats are substantially parallel to each other, and the parallelism is at least approximately kept by folding and curving the casing 12, but it is meant that it could include seats in several rows placed sided by side, and in case curved rows, so as to be able to assume more complex shapes.

Then, said plurality of external walls 17 implement a corresponding plurality of seats connected to each other, which receive a respective sensor 11, 11' and, thanks to the flexibility of the material constituting the casing 12, the relative position between the sensors 11, 11', or the shape of the detecting device 10, can be varied, or adapted to the shape of an irregular, and variable over time, contact surface as the human or animal body can be.

In the first example (figure 2A), the first sensor 11' which is wholly shielded, is arranged centrally with respect to the second sensors 11, so that, apart from the physical shielding, due to the second shielding layer 13', it could benefit from a geometrical shielding given by the presence of the first sensors 11 placed next to it.

Moreover, according to this second embodiment, next to the second sensor 11', there are two first sensors 11 in the seats of the casing 12 to its right and two first sensors 11 in the seats of the casing 12 to its left; such configuration allows to make use of the symmetry of the device 1 for possible procedures of filtering the interferences due to the radiations coming from the surrounding environment.

Alternatively, according to the second example (figure 2B), the first sensor 11', which is fully shielded, is arranged at an end of the casing 12, whereas the second sensors 11 form one single set of contiguous sensors, but it is meant that different configurations could be implemented to meet contingent needs.

In the embodiment examples of figures 2A, 2B, the detecting device 10 comprises five scintillation sensors 11, 11' in total, which produce a receiving surface with a width of 12-13 cm, but it is meant that such width can vary by increasing or decreasing the number and the sizes of the single scintillation sensors 11, 11'.

Each scintillation sensor 11, 11' comprises one scintillator 14, that is one element made of a material, usually a suitably doped crystalline or plastic material, which is capable of emitting light pulses, generally visible light or in the field of the ultraviolet radiations, which can be detected and counted.

Such emission is caused by the fact that such material is crossed by an incident ionizing radiation, that is high energy photons or charged particles, like indeed the gamma radiations produced by the decay of ⁴⁰K.

Thanks to the type of used scintillator, said sensors 11, 11' are capable of detecting radiations between 20 keV and 2000 keV.

Moreover, the sensor comprises a photomultiplier 15 (figure 3), for example made of suitably doped silicon converting the light signal produced by the scintillator 14 into an electric signal.

The above-mentioned photomultipliers 15 are connected by an electrical connection 16 which provides, at suitable terminals, a pulsed electric signal which can be suitably converted to represent the observed decays which are substantially proportional to the amount of existing Potassium, according to known formulations.

The photomultipliers 15 of each sensor are connected to a corresponding plurality of analog-to-digital converters, which are connected to a microprocessor, with at least a power supply system and a connecting electric circuit. The microprocessor will contain a memory with a software arranged for processing the data obtained from the sensors, that is the signals obtained through analog to digital converters.

Preferably, the power supply system comprises a rechargeable or disposable battery.

A transmitting, powered too, device could be further provided.

Preferably, the transmitter comprises a Bluetooth^{®} or Wi-fi^{®} antenna allowing the real-time transmission of the data detected by the detecting device 10. In this way, it is possible to monitor constantly the course of the electromagnetic radiations emitted by the decay of isotope ⁴⁰K and to operatively intervene should outlies for the application field of the device 10 be detected.

On this regard, in the examples of figures 2A, 2B, the detecting device 10, apart from the five scintillation sensors 11, 11' even comprises a service container 18 containing the transmitter, the plurality of analog to digital converters, the power supply system, the electric circuit and the microprocessor. Said service container 18 has a cylindrical shape similar to the one of the seats of the sensors.

In this way the shape and size of the detecting device 10 remain substantially unaltered, by guaranteeing the deformability and compactness features typical of the device 10 the invention relates to.

Advantageously, said first sensor 11' wholly covered with a first shielding layer 13' is used as reference sensor 11' for the background noise. In fact, despite the shielding of the second sensors 11, the device 10 will detect a small amount of radiations emitted by ⁴⁰K existing in the surrounding environment.

The noise deriving on the side of the contact surface, for example the human organism, could be removed by calibrating the device so as to detect only energy around of gamma radiations of ⁴⁰K, then of 1460 keV, so that the signal is affected as little as possible by the interferences due to the surrounding world.

The detecting device 10 can be calibrated so as to detect two energy windows: one between 10 and 400 KeV, and the other one energy around 1460 keV, in case by using different scintillator materials inside the single sensor 11, 11'.

Advantageously, the detecting device 10 is capable of detecting at least indirectly even the emissions β of ⁴⁰K which, by interacting with the tissue in which they are emitted, produce an electromagnetic radiation called braking radiation.

Then, the beta emissions and gamma emissions produced by ⁴⁰K can be detected separately, and compared to check the reliability of the obtained measurement, since the ratio between the number of emissions of one type and of the other type is substantially constant.

The device 10 the invention relates to even comprises a system for fastening to the source surface, comprising for example pockets, wearable bands or belts with buckles or other fastening systems.

Preferably, the fastening system comprises an elastic band comprising housings for the device 10; said band is equipped with a Velcro closure, to adjust the adhesion around the patient's bust.

Alternatively, the fastening system is a device of bandage type.

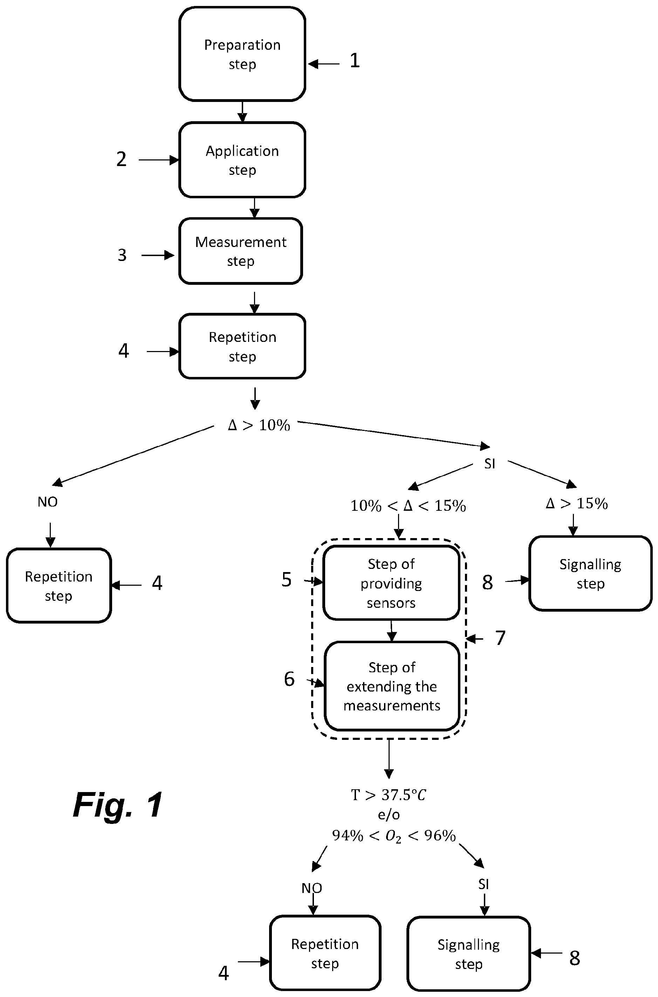

In a second aspect of the invention, with reference to figure 1, by way of example, a method for monitoring the Potassium concentration is described according to an application of clinical type, wherein the Potassium level in a living being is detected and monitored, in particular in an organ of a human being such as the heart or the upper lobe of the liver, which constitutes a source with in case irregular shape.

The liver is one of the organs containing higher amounts of Potassium, approximately on the average 4.5 grams in an adult person. Moreover, it is not subjected to intrinsic variations in its composition and then, against maintaining general homeostasis of the electrolytic equilibrium, the amount of hepatic Potassium remains stable over time, differently from what takes place in other organs and tissues such as kidney, muscles or bones.

Moreover, the positioning of the detecting device 10 at the liver level, allows a better use thereof, since the measurements that can be assumed by way of example of the detecting device can be of 18 cm x 10 cm, which correspond approximately to the hepatic aia. At last, the position liver adjacent to the front abdominal wall, that is near epidermis, increases the detecting capability of the device 10.

Another organ which can be conveniently monitored by the detecting device 10 within the present method is the heart. The heart overall holds about 0.72 g of Potassium, that is less than liver, but however a sufficient amount to detect alterations thereof with a detection of isotope ⁴⁰K.

The heart is an organ extremely sensitive to the electrolytic alterations and then of Potassium, which can easily cause arrhythmias, fibrillations until even lethal consequences.

Therefore, the fact of positioning the device 10 at the cardiac level could have a life-saving potential, since it would allow to identify early signs of alterations in the cardiac conduction, capable of generating fatal arrhythmias.

Within the field of infective pathologies such as COVID-19 pathology, affecting more severely the most fragile patients, that is those with previous cardiological complications or base pressure imbalances (hypertension) or in poly-pharmacological therapy which can cause alterations of Potassium at the level of cardiac cells, the detecting device can monitor the course of the disease itself, that is the safety level of the prescribed drugs, and identify in advance adverse events or fatal complications.

Therefore, based upon the patient complexity, one can decide to place the detecting device 10 at the level of the liver and/or heart.

Preferably, said method is applied to patients with electrolytical imbalances and before adhering the detecting device 10 to the patient it is good to know his/her clinical state and to know possible cardiac comorbidities.

Should said cardiac comorbidities be present, the detecting device 10 preferably is placed near the heart, alternatively near the liver.

Such method comprises a preparation step 1 wherein at least a device 10 detecting electromagnetic radiations emitted by the decay of Potassium isotope ⁴⁰K is provided, comprising a plurality of scintillation sensors 11, 11', the detecting device 10 being deformable.

The method further comprises an application step 2 wherein the shape of the detecting device 10 is adapted to an emitting surface with irregular shape of said source, and a measurement step 3 wherein a measurement of the Potassium concentration is performed, by obtaining it from the amount of electromagnetic radiations emitted by the decay of Potassium isotope ⁴⁰K.

Preferably, said method even comprises monitoring, wherein said measurement of the Potassium concentration is repeated periodically, such measurement checking if the deviation from the preceding measurement is lower or higher than 10%.

On this regard, a repetition step 4 can be provided, mainly consisting of repeating the measurement of the Potassium concentration by verifying, with respect to the initial measurement of the preceding step, if the deviation from the preceding measurement is lower or higher than a threshold value, preferably 10%.

Such value can define a slight variation in the Potassium basal level in the monitored organ.

In case the deviation from the preceding measurement of the Potassium concentration is lower than 10%, one continues to repeat periodically the measurement of the Potassium concentration.

In case the deviation from the preceding measurement of the Potassium concentration is higher than 10%, after the repetition step 4, one can activate an extension step 7, and/or activate a step 8 for signalling to the patient and/or to a health professional following the patient (figure 1).

In case the deviation from the preceding measurement of the Potassium concentration is comprised between 10% and 15%, the extension step 7 consists in a procedure for supplying sensors 5, wherein a temperature sensor (thermometer) and a sensor for the oxygen saturation (oximeter) are made available to the patient, which sensors are often used even in home monitoring, and in an extension step of measurements 6 consisting in measuring the body temperature and the oxygen saturation.

If the temperature is higher than a threshold value, which for an adult patient could be of 37.5°C, and if the oxygen saturation in blood will result comprised between 94% and 96%, it will be necessary to go from the above-mentioned signalling step 8, which produces an alarm which could be sent even to a medical management unit or general practitioner medical staff for their appropriate evaluations and for a possible hospitalization of the patient.

In the same way, for moderate Potassium variations, for example defined as a variation of 20% from the basal level, but without other alterations of the parameters, an alert message is sent to the patient and, in case of worsening, to the medical management unit or general practitioner medical staff for their appropriate evaluations and for a possible hospitalization of the patient.

Otherwise, should alarms of any type be not required, it is possible to return to monitoring, that is to the above-mentioned repetition step 4.

In case the deviation from the preceding measurement of the Potassium concentration is higher than an increased threshold value with respect to the basal level, preferably 15% for more safety, the above-mentioned signalling step 8 can be activated preferably electronically through an application which can be installed on a portable or fixed computer, a smartphone or a tablet, connected directly to the detecting device.

The monitoring step could be useful for evaluating periodically the Potassium level in an even different source, by observing the course of the emitted radiations over time.

It can be understood that an advantage of the monitoring method lies in the fact of allowing the remotely prolonged monitoring of the Potassium concentration. Said monitoring can take place during the regular carrying out of daily activities through said wearable and portable device.

Another advantage of said monitoring method, according to the described preferred embodiment, consists in the timely communication of the changed clinical conditions of the patient, both to the patient himself/herself, and to the health professional treating him/her. This rapidity in detecting and communicating the clinical situation allows a timely intervention by the health professional.

An additional advantage is that said monitoring method can be applied to patients with infective pathologies involving potentially severe cardiac implications, for which home isolation is required, for example the Covid-19 patients. In this case, performing the remote monitoring, reducing the health costs and not overloading the sub-intensive healthcare facilities.

## Claims

1. A detecting device (10) for monitoring the Potassium concentration, comprising:
• a plurality of scintillation sensors, each comprising at least one scintillator (14) and a relative photomultiplier (15), arranged to detect electromagnetic radiations between 20 KeV and 2000 Kev emitted by the decay of isotope ⁴⁰K, which consists of a first scintillation sensor (11'), which is fully enclosed in a first shielding layer (13'), and of second scintillation sensors (11), which are partially shielded with a second shielding layer (13) so as to shield a not receiving portion thereof and to leave uncovered a receiving portion thereof; and
• a casing (12) wherein a plurality of seats for scintillation sensors is formed, which are separate and distinct, but connected to each other, each seat for scintillation sensors receiving a respective scintillation sensor (11', 11) of said plurality of scintillation sensors, the casing (12) being deformable so that the position of each scintillation sensor (11, 11') could vary with respect to the position of others;
• a plurality of analog to digital converters, each one connected to a respective photomultiplier (15);
• at least a microprocessor and a respective memory with a software arranged for processing signals obtained through analog to digital converters; and
• at least a power supply system with a relative electric circuit,
wherein the receiving portions of said second scintillation sensors (11) are oriented so as to determine together a receiving surface of the detecting device (10), and wherein the casing (12) can be adapted to fit the shape of said receiving surface to an emitting surface with possibly irregular shape.

2. The detecting device (10) according to claim 1, wherein said seats and the scintillation sensors (11, 11') of said plurality of scintillation sensors have a respective substantially cylindrical shape, and wherein respective walls (17) of said seats conjugate to each other, by forming said deformable casing (12).

3. The detecting device (10) according to claim 3, wherein said plurality of analog to digital converters; said at least a microprocessor and a respective memory; and said at least a power supply system, with a relative electric circuit, are all contained in a service container (18) located in said casing (12).

4. The detecting device (10) according to claim 1, comprising a transmitter having at least an antenna, preferably operating by means of a Bluetooth^{®} or Wi-fi^{®} protocol, which is arranged to transmit in real time data detected by the detecting device (10).

5. The detecting device (10) according to any one of the preceding claims, which comprises a fastening system, arranged for connecting the detecting device (10) to a source.

6. The detecting device (10) according to claim 5, wherein the fastening system is an elastic band adjustable in length and at least a housing for the detecting device (10).

7. The detecting device (10) according to claim 5, wherein the fastening system comprises a bandage.

8. The detecting device (10) according to any one of the preceding claims, wherein the scintillation sensors (11, 11') are arranged to receive emissions of electromagnetic radiations with energy comprised between 10 and 400 KeV and energy around 1460 keV, in case by using different scintillator materials inside the single scintillation sensor (11, 11').

9. A method of using a device according to any of claims 1 - 8 to monitor the Potassium concentration of a source comprising the following steps:
• a preparation step (1) wherein the device (10) for detecting electromagnetic radiations emitted by the decay of Potassium isotope ⁴⁰K is provided;
• an application step (2) wherein the shape of the detecting device (10) is adapted to an emitting surface with possibly irregular shape of said source, so as to establish a correlation between the emitting surface and a capture area of the detecting device (10); and
• a measurement step (3) wherein a measurement of the Potassium concentration is performed, by obtaining it from the amount of electromagnetic radiations emitted by the decay of Potassium isotope ⁴⁰K.

10. The method for monitoring the Potassium concentration according to claim 9, wherein the scintillation sensors (11, 11') are arranged to receive emissions of electromagnetic radiations with energy comprised between 10 and 400 KeV and energy around 1460 keV, in case by using different scintillator materials inside the single scintillation sensor (11, 11').

## Patentansprüche

1. Detektionsvorrichtung (10) zur Überwachung der Kaliumkonzentration, aufweisend:
• Eine Mehrzahl von Szintillationssensoren, die jeweils aufweisen mindestens einen Szintillator (14) und einen zugehörigen Photomultiplier (15), die so angeordnet sind, dass sie elektromagnetische Strahlung zwischen 20 KeV und 2000 KeV erfassen, die durch den Zerfall des Isotops ⁴⁰K emittiert wird, bestehend aus einem ersten Szintillationssensor (11'), der vollständig in einer ersten Abschirmschicht (13') eingeschlossen ist, und zweiten Szintillationssensoren (11), die teilweise mit einer zweiten Abschirmschicht (13) abgeschirmt sind, um einen nicht empfangenden Teil davon abzuschirmen und einen empfangenden Teil davon unbedeckt zu lassen; und
• ein Gehäuse (12), in dem mehrere Sitze für Szintillationssensoren ausgebildet sind, die voneinander getrennt und unterschiedlich, aber miteinander verbunden sind, wobei jeder Sitz für Szintillationssensoren einen entsprechenden Szintillationssensor (11', 11) der mehreren Szintillationssensoren aufnimmt, wobei das Gehäuse (12) verformbar ist, so dass die Position jedes Szintillationssensors (11, 11') in Bezug auf die Position der anderen variieren kann;
• eine Mehrzahl von Analog-Digital-Wandlern, von denen jeder mit einem entsprechenden Photomultiplier (15) verbunden ist;
• mindestens einen Mikroprozessor und einen entsprechenden Speicher mit einer Software, die zur Verarbeitung der über Analog-Digital-Wandler erhaltenen Signale ausgebildet ist; und
• mindestens ein Stromversorgungssystem mit einem entsprechenden Stromkreis,
wobei die Empfangsabschnitte der zweiten Szintillationssensoren (11) so ausgerichtet sind, dass sie zusammen eine Empfangsfläche der Detektionsvorrichtung (10) bilden, und wobei das Gehäuse (12) angepasst werden kann, um die Form der Empfangsfläche an eine Emissionsfläche mit möglicherweise unregelmäßiger Form anzupassen.

2. Detektionsvorrichtung (10) gemäß Anspruch 1, wobei die Sitze und die Szintillationssensoren (11, 11') der Mehrzahl von Szintillationssensoren jeweils eine im Wesentlichen zylindrische Form aufweisen, und wobei jeweilige Wände (17) der Sitze durch Bildung des verformbaren Gehäuses (12) miteinander konjugieren.

3. Detektionsvorrichtung (10) gemäß Anspruch 3, wobei die mehreren Analog-Digital-Wandler, der mindestens eine Mikroprozessor und ein entsprechender Speicher sowie das mindestens eine Stromversorgungssystem mit einem entsprechenden Stromkreis, alle in einem Servicebehälter (18) enthalten sind, der sich in dem Gehäuse (12) befindet.

4. Detektionsvorrichtung (10) gemäß Anspruch 1, aufweisend einen Transmitter, der mindestens eine Antenne aufweist, vorzugsweise mittels eines Bluetooth^{®}- oder Wi-Fi^{®}-Protokolls arbeitet und so eingerichtet ist, dass er in Echtzeit Daten, die von der Detektionsvorrichtung (10) erfasst wurden, überträgt.

5. Detektionsvorrichtung (10) gemäß einem der vorstehenden Ansprüche, aufweisend ein Befestigungssystem, das zum Verbinden der Detektionsvorrichtung (10) mit einer Quelle ausgebildet ist.

6. Detektionsvorrichtung (10) gemäß Anspruch 5, wobei das Befestigungssystem ein elastisches Band ist, das in der Länge verstellbar ist, und mindestens ein Gehäuse für die Detektionsvorrichtung (10).

7. Detektionsvorrichtung (10) gemäß Anspruch 5, wobei das Befestigungssystem eine Bandage aufweist.

8. Detektionsvorrichtung (10) gemäß einem der vorstehenden Ansprüchen, wobei die Szintillationssensoren (11, 11') so arrangiert sind, dass sie Emissionen elektromagnetischer Strahlung mit einer Energie zwischen 10 und 400 KeV und Energie um 1460 keV empfangen, im Fall von Verwendung verschiedener Szintillatormaterialien im einzelnen Szintillationssensor (11, 11').

9. Verfahren zur Verwendung einer Vorrichtung gemäß einem der Ansprüche 1 bis 8 zur Überwachung die Kaliumkonzentration von einer Quelle, aufweisend die folgenden Schritte:
• einen Vorbereitungsschritt (1), in dem die Vorrichtung (10) zum Erfassen elektromagnetischer Strahlung, die durch den Zerfall des Kaliumisotops ⁴⁰K emittiert wird, bereitgestellt wird;
• einen Anwendungsschritt (2), bei dem die Form der Detektionsvorrichtung (10) an eine emittierende Oberfläche mit möglicherweise unregelmäßiger Form der Quelle angepasst wird, um eine Korrelation zwischen der emittierenden Oberfläche und einem Erfassungsbereich der Detektionsvorrichtung (10) zu etablieren; und
• einen Messschritt (3), in dem eine Messung der Kaliumkonzentration durchgeführt wird, indem sie aus der Menge der elektromagnetischen Strahlung ermittelt wird, die durch den Zerfall des Kaliumisotops ⁴⁰K emittiert wird.

10. Verfahren zur Überwachung der Kaliumkonzentration gemäß Anspruch 9, wobei die Szintillationssensoren (11, 11') so angeordnet sind, dass sie Emissionen von Elektromagnetische Strahlung mit einer Energie zwischen 10 und 400 KeV und Energie um 1460 KeV empfangen, im Fall von Verwendung verschiedener Szintillatormaterialien im einzelnen Szintillationssensor (11, 11').

## Revendications

1. Dispositif de détection (10) pour surveiller la concentration de potassium, comprenant :
• une pluralité de capteurs à scintillation, comprenant chacun au moins un scintillateur (14) et un photomultiplicateur relatif (15), conçus pour détecter les rayonnements électromagnétiques entre 20 KeV et 2000 Kev émis par la désintégration de l'isotope ^{40K}, qui se compose d'un premier capteur à scintillation (11'), qui est entièrement enfermé dans une première couche de protection (13'), et de deuxièmes capteurs à scintillation (11), qui sont partiellement protégés par une deuxième couche de protection (13) de manière à protéger une partie qui ne les reçoit pas et à laisser à découvert une partie qui les reçoit ; et
• un boîtier (12) dans lequel sont formés plusieurs sièges pour capteurs de scintillation, séparés et distincts, mais reliés les uns aux autres, chaque siège pour capteurs de scintillation recevant un capteur de scintillation respectif (11', 11) de ladite pluralité de capteurs de scintillation, le boîtier (12) étant déformable de sorte que la position de chaque capteur de scintillation (11, 11') puisse varier par rapport à la position des autres ;
• plusieurs convertisseurs analogiques-numériques, chacun connecté à un photomultiplicateur (15) ;
• au moins un microprocesseur et une mémoire respective avec un logiciel conçu pour traiter les signaux obtenus par les convertisseurs analogiques-numériques ; et
• au moins un système d'alimentation avec un circuit électrique relatif,
dans lequel les parties réceptrices desdits deuxièmes capteurs à scintillation (11) sont orientées de manière à déterminer ensemble une surface réceptrice du dispositif de détection (10), et dans lequel le boîtier (12) peut être adapté à la forme de ladite surface réceptrice à une surface émettrice dont la forme peut être irrégulière.

2. Dispositif de détection (10) selon la revendication 1, dans lequel lesdits sièges et les capteurs de scintillation (11, 11') de ladite pluralité de capteurs de scintillation ont une forme sensiblement cylindrique respective, et dans lequel les parois respectives (17) desdits sièges se conjuguent l'une à l'autre, en formant ledit boîtier déformable (12).

3. Dispositif de détection (10) selon la revendication 3, dans lequel ladite pluralité de convertisseurs analogiques-numériques, ledit au moins un microprocesseur et une mémoire respective, et ledit au moins un système d'alimentation, avec un circuit électrique relatif, sont tous contenus dans un conteneur de service (18) situé dans ledit boîtier (12).

4. Dispositif de détection (10) selon la revendication 1, comprenant un émetteur ayant au moins une antenne, fonctionnant de préférence au moyen d'un protocole Bluetooth^{®} ou Wi-fi^{®}, qui est agencé pour transmettre en temps réel les données détectées par le dispositif de détection (10).

5. Dispositif de détection (10) selon l'une quelconque des revendications précédentes, qui comprend un système de fixation, agencé pour connecter le dispositif de détection (10) à une source.

6. Dispositif de détection (10) selon la revendication 5, dans lequel le système de fixation est une bande élastique réglable en longueur et au moins un boîtier pour le dispositif de détection (10).

7. Dispositif de détection (10) selon la revendication 5, dans lequel le système de fixation comprend un bandage.

8. Dispositif de détection (10) selon l'une quelconque des revendications précédentes, dans lequel les capteurs à scintillation (11, 11') sont disposés pour recevoir des émissions de radiations électromagnétiques avec une énergie comprise entre 10 et 400 KeV et une énergie autour de 1460 keV, dans le cas où l'on utilise différents matériaux de scintillation à l'intérieur du capteur à scintillation unique (11, 11').

9. Méthode d'utilisation d'un dispositif selon l'une des revendications 1 à 8 pour surveiller la concentration en potassium d'une source , comprenant les étapes suivantes :
- une étape de préparation (1) au cours de laquelle le dispositif (10) de détection des radiations électromagnétiques émises par la désintégration de l'isotope ^{40K}du potassium est fourni ;
- une étape d'application (2) dans laquelle la forme du dispositif de détection (10) est adaptée à une surface émettrice de forme éventuellement irrégulière de ladite source, de manière à établir une corrélation entre la surface émettrice et une zone de capture du dispositif de détection (10) ; et
- une étape de mesure (3) dans laquelle on mesure la concentration de potassium, en l'obtenant à partir de la quantité de radiations électromagnétiques émises par la désintégration de l'isotope ⁴⁰K du potassium.

10. Méthode de surveillance de la concentration de potassium selon la revendication 9, dans laquelle les capteurs à scintillation (11, 11') sont agencés pour recevoir des émissions de radiations électromagnétiques avec une énergie comprise entre 10 et 400 KeV et une énergie autour de 1460 keV, dans le cas où l'on utilise différents matériaux de scintillation à l'intérieur du capteur à scintillation unique (11, 11').
